# EUROPÄISCHE PATENTANMELDUNG

(11) **EP 0 713 764 A2**
(43) Veröffentlichungstag der Anmeldung: **29.05.1996**
(21) Anmeldenummer: 95117654.4
(22) Anmeldetag: 09.11.1995
(51) Int. Cl.: B32B 27/32

(54) **Mehrschichtige, rollneigungsarme, mattierte Polyolefinfolie zur Verwendung als Trägerfilm für Wundpflaster**

(30) Priorität: 22.11.1994 DE 4441416
(71) Anmelder: WOLFF WALSRODE AG, D-29655 Walsrode (DE)
(72) Erfinder: Schultze, Dirk, Dr., D-29683 Fallingbostel (DE); Reiners, Ulrich, Dr., D-29643 Neuenkirchen (DE)
(74) Vertreter: Braun, Rolf, Dr.

(57) **Zusammenfassung**

Die vorliegende Erfindung betrifft mindestens dreischichtige, beidseitig matte Folie mit geringer Rollneigung, wobei daß die Folie durch Coextrusion hergestellt wurde und wobei die Aussenschichten der Folie mindestens zwei verschiedene Polymerharze enthalten, wobei ein Harz ein Polyethylen oder Ethylencopolymerisat mit niedrigem Kristallinitätsgrad und einem Schmelzflußindex von 1 g/10 min bis 6 g/10min ist, gemessen nach DIN 53 735 bei 190°C mit einer Prüfbelastung von 2,16 kg, und das zweite Polyolefinharz ein Propylenpolymerisat mit einem Schmelzflußindex von 1 g/10 min bis 6 g/10 min ist, gemessen nach DIN 53 735 bei 230°C und einer Prüfbelastung von 2,16 kg.

## Beschreibung

Die Erfindung bezieht sich auf rollneigungsarme, mehrschichtige, mindestens dreischichtige Polyolefinfolien, die sich durch Coextrusion herstellen lassen. Es werden Rezeptur-bedingte matte Strukturen erhalten, d.h. ohne weitere Behandlungsschritte werden in-line matte Folien erzeugt. Die Folien werden aus Olefinpolymeren oder auch Olefin-Copolymeren aufgebaut und können Anteile an Verarbeitungshilfsmitteln, Farbpigmenten und Stabilisatoren enthalten. Durch ihre Oberflächencharakteristik eignen sich diese Folien besonders für die Verwendung als Trägerfilme von Wundpflastern, Heftpflastern oder Pflaster-Strips.

Für den Einsatz der erfindungsgemäßen Folien bieten sich vielfältige Anwendungsmöglichkeiten. Hier soll aber vor allem auf die Verwendung als Trägerfilme von Wundabdeckungen abgehoben werden. Folien für derartige Anwendungen müssen eine Vielzahl von Anforderungen erfüllen, die neben den technischen vor allem ästhetische Bedingungen beinhalten.

Folien für medizinische Pflaster müssen formstabil sein, sich chemisch oder physikalisch vorbehandeln lassen, um bedruckt und/oder beschichtet zu werden. Sie sollen zudem mechanische Eigenschaften besitzen, die eine geringe Rollneigung, eine Mindest-Steifigkeit und Mindest-Bruchkräfte umfassen. Die Bruchkräfte sollten beispielsweise >20 N/15mm für eine 80 µm dicke Folie sein. Die geringe Rollneigung stellt ein verarbeitungs- und anwendungtechnisches Erfordernis dar. Bei ausgeprägter Rollneigung können klebstoffbeschichtete Teile der Pflaster gegeneinander verkleben.

Zu den ästhetischen Anforderungen gehört in erster Linie eine seidig-matte Oberfläche, die ein hautähnliches Erscheinungsbild gewährleistet. Bei transparenten Folien scheint die Haut i.a. durch, so daß eine intensive Beeinflussung des Farbeindrucks durch die Folie unerwünscht ist. Soll eine Wundabdeckung verdeckt werden, so sind eingefärbte Typen im Einsatz. Allgemein verbreitet sind beige eingefärbte Pflasterfolien für diesen Einsatzzweck. Matte Oberflächen werden allgemein durch den Glanz beurteilt, minimale Glanzwerte werden angestrebt. Für Pflaster haben sich allgemein Glanzwerte < 5 bewährt, gemessen nach DIN 67530 unter einem Winkel von 20°.

Eine geringe Trübung ist von Vorteil, da so der Untergrund besser durchscheinen kann bzw. bei eingefärbten Folien die Farben eine höhere Brillianz besitzen. Auch die Haptik der Pflasterfolien muß ansprechend sein, hierzu zählt insbesondere ein weicher Griff. Der Griff wird neben den mechanischen Eigenschaften durch die Oberflächencharakteristik der Folie mitbestimmt.

Die Mattierungsverfahren, die zum Stand der Technik gehören, umfassen vor allem Präge- und Beschichtungstechniken, bei denen die Folie i.a. an gravierten oder anderweitig mattierten Walzen vorbeigeführt wird und unter Druck die gewünschte Oberflächenstruktur erhält. Diese Technik unterliegt jedoch gewissen Beschränkungen. Es ist von besonderem wirtschaftlichen Interesse, daß Prägelinien nur mit geringen Geschwindigkeiten betrieben werden können, die deutlich niedriger als die der Folienherstellungsanlagen liegen. Zudem sind der Prägung durch die Relaxation der auf die zu prägenden Materialien gepressten Strukturen und die Gravurtiefen Grenzen gesetzt. Matt-beschichtete Folien neigen zu einer deutlichen Rollneigung, die vor allem quer zur Bahnrichtung nicht durch eine Anpassung von Verfahrensparametern wie Vor- oder Bahnspannung begrenzt werden kann.

Verschiedentlich sind in der Patentliteratur auch in-line-Verfahren zur Mattierung erwähnt. Die japanische Patentschrift 3216-722 (Mitsubishi Petrochemicals) beschreibt Mischungen aus Ethylen-Vinylacetat-Copolymeren (EVA) und Ethylen-alpha-Olefin-Copolymeren zur in-line-Mattierung. Mit solchen Mischungen lassen sich aber nicht die für medizinische Pflaster geforderten geringen Glanzwerte erzielen.

In EP 0 508 415 (Nippon Petrochemicals) werden Mischungen aus Propylen-Polymerisaten und Ethylen-alpha-Olefin-Copolymerisaten beschrieben. Diese auf Propylen basierenden Materialien sind zu steif um als anschmiegsames Pflaster Verwendung zu finden.

Die EP 0 547 834 und die EP 0 235 930 (beide Minnesota Mining & Manufacturing) beschreiben Blends aus mesophasigem oder isotaktischem Polypropylen und Mischungskomponenten. Die so erhaltenen Folien sind aber wiederum zu steif, um die Anforderungen der oben beschriebenen medizinischen Pflasteranwendung zu erfüllen.

Blends aus EVA und elastomeren Ethylen-Propylen-Copolymerisaten werden in der BE 899-649 (Grace) beschrieben. Diese Folien sind wiederum zu elastisch, sie besitzen eine zu geringe Reißfestigkeit.

Allen bekannten in-line mattierten Einschicht-Folien auf der Basis von Blendrezepturen ist zudem eigen, daß sie aufgrund der Entmischungsstruktur geringe Festigkeiten oder Bruchdehnungen besitzen, die durch die Schwächung des Materialgefüges bedingt sind.

In der EP 0 459 059 (Minnesota Mining & Manufacturing) werden Mischungen von EVA in einer kontinuierlichen Phase aus isotaktischem PP beschrieben. Das EVA dient hier explizit der Störung des Materialgefüges und der damit verbundenen Erhöhung der Wasserdampfdurchlässigkeit durch auftretende Mikroporen. Eine Mattierung der Folienoberfläche wird dort nicht beschrieben.

Ein matter, mehrschichtiger Aufbau wird in der US 4,880,682 (Exxon) beschrieben. Dieser besteht aus zwei weichen EVA-Aussenschichten und einer elastischen Kautschuk-Kernschicht. Wird die Folie gedehnt, so verliert sie ihre Mattigkeit, da die durch Stauchung der Aussenschichten erzeugten Dickenunterschiede hinfällig werden. Außerdem besitzt die Folie aufgrund des Kautschuk-Folienkerns keine ausreichende Steifigkeit. Bei Verwendung von Trennpapieren zur Abdeckung des Pflasterklebstoffes ist das Abtrennen des Trennmaterials dadurch erschwert.

Es galt die Aufgabe zu lösen, eine rollneigungsarme, matte Folie bereitzustellen, die ihren matten Charakter intrinsisch durch ihre Rezeptur erhält und somit keiner mechanischen Nachbearbeitung bedarf. Sie sollte einen Glanzwert von maximal 5, gemessen nach DIN 67530 unter einem Winkel von 20°, aufweisen. Die Trübung der erfindungsgemäßen Folien sollte, soweit sie transparent ausgeführt werden, nicht über 70 % liegen, gemessen nach ASTM D 1003. Nur bei minimaler Trübung der Folie kann der von der Folie bedeckte Untergrund durchscheinen oder die mögliche Einfärbung optimal d.h. ungetrübt zur Geltung kommen. Die Trübung kann jedoch nur an nicht eingefärbten Produkten bestimmt werden.

Ihre Elastizität sollte zudem so hoch sein, daß sie sich gut an Bewegungen der durch Pflaster abgedeckten Körperteile anpassen kann. Zudem sollte sie jedoch so steif sein, daß sie sich auf den gängigen Verarbeitungsmaschinen wie Stanzen, Perforiermaschinen, Kaschieranlagen gut einsetzen, wickeln und handhaben läßt. Zur Gewährleistung einer guten Haftklebstoffverankerung sollte sie sich chemisch und/oder physikalisch vorbehandeln lassen.

Die Aufgabe wurde durch coextrudierte, mehrschichtige Folien der eingangs erwähnten Gattung basierend auf Mischungen aus Olefinpolymerisaten gemäß Anspruch 1 gelöst.

Die Erfindung umfasst mindestens dreischichtige, aufgrund ihrer Rezeptur intrinsich beidseitig mattierte Folien, wobei die Aussenschichten der Folien im wesentlichen mindestens zwei verschiedene olefinische Polymerisate enthalten. Die eingeschlossene Schicht bzw. die eingeschlossenen Schichten bestehen bevorzugt aus nur einem Olefin-Polymerisat. Erstaunlich war, daß durch den dreischichtigen Aufbau der Folie die Rollneigung minimiert werden kann.

Für die Kernschicht der erfindungsgemäßen Folien werden bevorzugt Ethylenpolymerisate und/oder Ethylencopolymerisate mit niedrigem Kristallinitätsgrad verwendet, deren Schmelzflußindex in einem Bereich von 1 g/10 min bis 6 g/10min, gemessen nach DIN 53 735 bei 190°C und einer Prüfbelastung von 2,16 kg, liegt. Besonders bevorzugt sind hierbei Sauerstoffatome enthaltende Ethylencopolymerisate mit einem Comonomerrestanteil von 2 Gew.-% bis 10 Gew.-%, jeweils bezogen auf die für die Mittelschicht eingesetzte Gesamtpolymermasse.

Durch den bevorzugten Einsatz nur einer olefinischen Komponente für die Mittelschicht gelingt es, die eingeschlossene Schicht bzw. die eingeschlossenen Schichten als festigkeitstragende Einheit in den Folienaufbau zu integrieren.

Die aus mindestens zwei unterschiedlichen Polymerharzen bestehenden Aussenschichten der erfindungsgemäßen Folien, enthalten zumindest ein Harz aus der Gruppe der Polyethylene und Ethylencopolymerisate mit niedrigem Kristallinitätsgrad. Die erfindungsgemäß bevorzugten Harze aus dieser Gruppe besitzen Schmelzflußindices von 1 g/10 min bis 6 g/ 10min, gemessen nach DIN 53 735 bei 190°C mit einer Prüfbelastung von 2,16 kg. Bevorzugt sind Sauerstoffatome enthaltende Ethylencopolymerisate mit Massenanteilen an der Aussenschicht zwischen 60 Gew.-% und 85 Gew.-%. Zumindest ein weiteres Polyolefinharz ist ein Propylenpolymerisat mit einem Schmelzflußindex von 1 g/10 min bis 6 g/10 min, gemessen nach DIN 53 735 bei 230°C und einer Prüfbelastung von 2,16 kg. Bevorzugt sind Anteile von 15 Gew.-% bis 40 Gew.-% an Propylenhomopolymerisaten in der Aussenschicht.

Nicht naheliegend war es, daß derart zusammengesetzte Kunststoffmischungen ausgeprägt matte Oberflächen besitzen, die durch Glanzwerte < 5 gekennzeichnet sind, gemessen nach DIN 67 530 unter einem Winkel von 20°. Der geringe Glanz geht mit Rauhtiefen der Oberfläche von einigen µm einher.

Erstaunlich war ebenso die gute Anbindung der Aussenschichten an die Kernschicht oder Kernschichten sowie das gleichförmige Fließverhalten der Schmelzeströme in den mindestens drei Schichten bei Verarbeitung mit Coextrusionswerkzeugen. Es traten keine Fließstrukturen beim Austritt der Schmelzefahne aus der Düse auf.

Der Versteifung der Folie durch das Einmischen des im Vergleich zu Ethylenpolymerisaten mit geringer Dichte steifen Propylenpolymerisats wird durch die Auswahl einer Matrix-Komponente für die Aussenschichten mit geringer Härte, d.h. hoher Flexibilität, entgegengewirkt.

In einer bevorzugten Ausführungsform weisen die Aussenschichten einen Anteil von 20-30 Gew.-% eines partiell isotaktischen Propylenhomopolymerisats auf. Letzteres besitzt eine Dichte zwischen 0,90 g/cm³ und 0,91 g/cm³ sowie einen Schmelzflußindex zwischen 2 g/10 min und 5 g/10 min, besonders geeignet sind Schmelzflußindices von 3 g/10 min bis 4 g/10 min, gemessen nach DIN 53 735 bei 230°C und einer Prüfbelastung von 2,16 kg. Das zweite Harz kommt aus der Gruppe der Ethylencopolymerisate, wobei Sauerstoffatome enthaltende Copolymerisate bevorzugt sind. Der Anteil des Ethylencopolymerisats liegt bei 70-80 Gew.-%, bezogen auf die Gesamtmasse der jeweiligen Aussenschicht. Für diese Komponente finden Materialien mit Dichten zwischen 0,93 g/cm³ und 0,97 g/cm³, einem Anteil an Comonomerresten von 5 Gew.-% bis 25 Gew.-%, bezogen auf die Gesamtmasse des für die jeweilige Aussenschicht verwendeten jeweiligen Ethylencopolymerisats, und einem Schmelzflußindex von 1 g/10 min bis 3 g/10min, gemessen nach DIN 53 735 bei 190°C und einer Prüfbelastung von 2,16 kg. Das die Mittelschicht bildende Polymerharz kommt aus der Gruppe der Ethylencopolymerisate und hat eine Dichte zwischen 0,91 g/cm³ und 0,94 g/cm³ sowie einen Schmelzflußindex von 1 g/10min bis 3 g/10 min, gemessen nach DIN 53 735 bei 190°C und einer Prüfbelastung von 2,16 kg. Die angegebenen Massenanteile der Polymerisate beziehen sich jeweils auf die Gesamtmasse der für die Herstellung der jeweiligen Aussenschicht eingesetzten Polymerrohstoffe.

In einer bevorzugten Ausführung können einzelne oder alle Schichten der erfindungsgemäßen mehrschichtigen Folie durch Zugabe von Verarbeitungshilfen, Füllstoffen, Farben und Stabilisatoren modifiziert sein. Farben ermöglichen die Herstellung von Pflastern für spezielle Einsatzgebiete.

In einer dieser ausgewählten Ausführungformen enthält mindestens eine der Schichten Farbpigmente. Beige eingefärbte Folie ist beliebt, wenn Wunde und Wundabdeckung nicht offen sichtbar sein sollen. Bunte Einfärbungen und Druckapplikationen finden vielfach Eingang in Kinderpflaster. Weitere Additive wie Silikate und Wachse modifizieren die anwendungstechnischen Eigenschaften, insbesondere das Gleitverhalten. Stabilisatoren ermöglichen es, die erfindungsgemäßen Folien über einen längeren Zeitraum zu erhalten bzw. Schädigungen bei der Verarbeitung zu vermeiden. Die gängigen Additive für Kunststoffe sind von Gächter und Müller im: Handbuch der Kunststoff Additive, Hanser Verlag, München 1983, beschrieben.

Die erfindungsgemäßen Folien besitzen besonders vorteilhafte Eigenschaften, wenn die Folie eine symmetrische Schichtdickenabfolge, bezogen auf den geometrischen Mittelpunkt der Folie, besitzt. Der jeweilige Anteil der einzelnen Aussenschichten an der Gesamtschichtdicke der Folie sollte zwischen 10 % und 40 % und der Anteil der Mittelschicht zwischen 20 % und 80 %, jeweils bezogen auf die Gesamtdicke der Folie, betragen. In einer besonders bevorzugten Ausführung sollte der Gesamtanteil der Aussenschichten, d.h. die Summe der Dickenanteile der Aussenschichten, an der Gesamtdicke der Folie nicht mehr als 50 % betragen. Bei streng symmetrischem Aufbau der Schichten hinsichtlich Schichtdicke und - rezeptur werden rollneigungsfreie Folien erhalten.

Die erfindungsgemäßen Folien lassen sich nach den gängigen Folien-Co-Extrusions-Verfahren herstellen. Als besonders geeignet für deren Herstellung hat sich der Blasfolienprozeß erwiesen. Hier wird die Schmelze von einer Ringdüse abgezogen, der erhaltene Schlauch i.a. durch einen gegenüber der Umgebung erhöhten Innendruck aufgeweitet, schließlich flachgelegt, abgequetscht, aufgetrennt und gewickelt.

Eine besondere Ausführungsform der erfindungsgemäßen Folien zeichnet sich dadurch aus, daß die Folie eine Gesamtschichtdicke zwischen mindestens 40 µm und höchstens 200 µm aufweist. Besonders geeignet sind Folien mit mindestens 50 µm und höchstens 100 µm Dicke.

Die erfindungsgemäßen Folien können in einer speziellen Ausführung ein- oder beidseitig wenigstens einem der bekannten chemischen und/oder physikalischen Oberflächen-Behandlungsverfahren unterzogen werden.

Im Hinblick auf ihre spätere Verwendung eignen sich hierzu ein oder mehrere der chemischen oder physikalischen Behandlungsverfahren zur Modifizierung der Oberflächeneigenschaften wie beispielsweise die Corona-, Plasma-, Fluor- aber auch Flammbehandlung. Solche Verfahren wurden beispielsweise von Dom und Wahono in Maschinenmarkt 96 (1990) 34-39 oder Milker und Möller in Kunststoffe 82 (1992) 978-981 ausführlich dargestellt. Ein besonders bevorzugtes Verfahren ist die Corona-Behandlung.

Bei der Corona-Behandlung wird zweckmäßigerweise so vorgegangen, daß die Folie zwischen zwei als Elektroden dienenden Leiterelementen hindurchgeführt wird, wobei zwischen den Elektroden eine so hohe Spannung - üblicherweise Wechselspannung von etwa 10 kV mit einer Frequenz von 10 kHz - anliegt, daß Sprüh- oder Coronaentladungen stattfinden können. Durch diese Entladungen wird die Luft entlang der Folienoberfläche ionisiert, so daß es zu Reaktionen an der Folienoberfläche kommt, bei denen im Vergleich zu der Polymermatrix polarere Gruppen entstehen. Die für die Vorbehandlung der erfindungsgemäßen Folien benötigten Behandlungsintensitäten liegen hierbei im üblichen Rahmen, wobei Behandlungsintensitäten bevorzugt sind, die Oberflächenspannungen von 38 - 60 mN/m ergeben.

Gegenstand der Erfindung ist ebenso die Verwendung der erfindungsgemäßen Folien als Trägerfilme in medizinischen Anwendungen. Durch ihre seidig-matte Oberfläche und ihren angenehmen Griff eignen sich die erfindungsgemäßen Folien für die Verwendung als Trägerfilme von medizinischen Pflasterfolien wie Rollenpflaster, Heftpflaster und Pflaster-Strips. Sie können sich sowohl flexibel den Bewegungen der Haut anpassen, aber sich auch gut vom Releasematerial abtrennen lassen.

Im folgenden wird die Erfindung anhand von Beispielen noch näher erläutert, ein Vergleich der Beispiele erfolgt in Tabelle 1.

### Beispiel 1

Mit einem Dreischicht-Blasfolienwerkzeug wurde eine dreischichtige Folie mit einer Nennschichtdickenabfolge 15 µm, 50 µm, 15 µm geformt. Die 50 µm dicke Mittelschicht der Folie wurde aus einem EVA-Copolymer mit einem VA-Anteil von 5 Gew.-%, bezogen auf die Gesamtmasse des für die Mittelschicht eingesetzten EVA-Copolymers, einem Schmelzflußindex (MFI) von 1,8 g/10 min, gemessen nach DIN 53 735 bei 190°C mit einer Prüfbelastung von 2,16 kg, und einer Dichte von 0,926 g/cm³ gebildet. Die beiden 15 µm dicken Aussenschichten der Folie wurden jeweils aus Mischungen unter Einsatz von 70 Gew.-% eines EVA-Copolymeren mit einem VA-Anteil von 18 Gew.-%, bezogen auf die eingesetzte Gesamtmasse EVA in den Aussenschichten, einem MFI von 2 g/10 min, gemessen nach DIN 53 735 bei 190°C mit einer Prüfbelastung von 2,16 kg, und einer Dichte von 0,926 g/cm³ sowie 30 Gew.-% eines Propylenhomopolymerisats mit einer Dichte von 0,905 g/cm³ und einem MFI von 3,3 g/10 min, gemessen nach DIN 53 735 bei 230°C mit einer Prüfbelastung von 2,16 kg, hergestellt.

### Beispiel 2

Mit einem Dreischicht-Blasfolienwerkzeug wurde eine dreischichtige Folie mit einer Nennschichtdickenabfolge 15 µm, 50 µm, 15 µm geformt. Die 50 µm dicke Mittelschicht der Folie wurde aus einem EVA-Copolymer mit einem VA-Anteil von 5 Gew.-%, einem Siliciumdioxid-Anteil von 1000 ppm, jeweils bezogen auf die Gesamtmasse des für die Mittelschicht verwendeten EVA-Copolymerisats, einem MFI von 1,8 g/10 min, gemessen nach DIN 53 735 bei 190°C mit einer Prüfbelastung von 2,16 kg, und einer Dichte von 0,926 g/cm³ gebildet. Die beiden 15 µm dicken Aussenschichten der Folie wurden jeweils aus Mischungen unter Einsatz von 75 Gew.-% eines EVA-Copolymeren mit einem VA-Anteil von 18 Gew.-%, bezogen auf die für die Aussenschichten eingesetzte Gesamtmasse EVA, einem MFI von 2 g/10 min, gemessen nach DIN 53 735 bei 190°C mit einer Prüfbelastung von 2,16 kg, und einer Dichte von 0,926 g/cm³ sowie 25 Gew.-% eines Propylenhomopolymerisats mit einer Dichte von 0,900 g/cm³ und einem MFI von 3,5 g/10 min, gemessen nach DIN 53 735 bei 230°C mit einer Prüfbelastung von 2,16 kg, hergestellt.

### Beispiel 3

Mit einem Dreischicht-Blasfolienwerkzeug wurde eine dreischichtige Folie mit einer Nennschichtdickenabfolge 10 µm, 50 µm, 10 µm geformt. Die 50 µm dicke Mittelschicht der Folie wurde aus einem EVA-Copolymer mit einem VA-Anteil von 5 Gew.-%, einem Siliciumdioxid-Anteil von 1000 ppm, jeweils bezogen auf die Gesamtmasse des für die Mittelschicht verwendeten EVA-Copolymerisats, einem MFI von 1,8 g/10 min, gemessen nach DIN 53 735 bei 190°C mit einer Prüfbelastung von 2,16 kg, und einer Dichte von 0,926 g/cm³ gebildet.

Die eine 10 µm dicke Aussenschicht der Folie wurde aus einer Mischung unter Einsatz von 60 Gew.-% eines EVA-Copolymeren, charakterisiert durch einen VA-Anteil von 18 Gew.-%, bezogen auf die eingesetzte Gesamtmasse des Copolymeren, einem MFI von 2 g/10 min, gemessen nach DIN 53 735 bei 190°C mit einer Prüfbelastung von 2,16 kg, und eine Dichte von 0,926 g/cm³, 25 Gew.-% eines Propylenhomopolymerisats, charakterisiert durch eine Dichte von 0,900 g/cm³ und einen MFI von 3,5 g/10 min, gemessen nach DIN 53 735 bei 230°C mit einer Prüfbelastung von 2,16 kg, sowie 15 Gew.-% eines EVA-Copolymeren, charakterisiert durch einen VA-Anteil von 5 Gew.-%, bezogen auf die eingesetzte Gesamtmasse des Copolymerisats, einen MFI von 1,8 g/10 min, gemessen nach DIN 53 735 bei 190°C mit einer Prüfbelastung von 2,16 kg, und eine Dichte von 0,926 g/cm³, gebildet. Diese Aussenschicht eignet sich bevorzugt für eine Oberflächenbehandlung.

Die zweite 10 µm dicke Aussenschicht der Folie wurde aus einer Mischung unter Einsatz von 55 Gew.-% eines EVA-Copolymeren, charakterisiert durch einen VA-Anteil von 18 Gew.-%, bezogen auf die eingesetzte Gesamtmasse des Copolymeren, einem MFI von 2 g/10 min, gemessen nach DIN 53 735 bei 190°C mit einer Prüfbelastung von 2,16 kg, und einer Dichte von 0,926 g/cm³, 25 Gew.-% eines Propylenhomopolymerisats, charakterisiert durch eine Dichte von 0,900 g/cm³ und einen MFI von 3,5 g/10 min, gemessen nach DIN 53 735 bei 230°C mit einer Prüfbelastung von 2,16 kg, 12 Gew.-% eines Ethylenpolymerisats, charakterisiert durch eine Dichte von 0,918 g/cm³ und einen MFI von 15 g/10 min, gemessen nach DIN 53 735 bei 190°C mit einer Prüfbelastung von 2,16 kg, sowie 8 Gew.-% Talkum zusammengesetzt.

### Beispiel 4

Mit einem Dreischicht-Blasfolienwerkzeug wurde eine dreischichtige Folie mit einer Nennschichtdickenabfolge 10 µm, 40 µm, 10 µm geformt. Die 40 µm dicke Mittelschicht der Folie wurde aus einem EVA-Copolymer mit einem VA-Anteil von 5 Gew.-%, einem Siliciumdioxid-Anteil von 1000 ppm, jeweils bezogen auf die Gesamtmasse des für die Mittelschicht verwendeten EVA-Copolymerisats, einem MFI von 1,8 g/10 min, gemessen nach DIN 53 735 bei 190°C mit einer Prüfbelastung von 2,16 kg, und einer Dichte von 0,926 g/cm³ gebildet.

Die eine 10 µm dicke Aussenschicht der Folie wurde aus einer Mischung unter Einsatz von 55 Gew.-% eines EVA-Copolymeren, 25 Gew.-% eines Propylenhomopolymerisats sowie 20 Gew.-% eines weiteren EVA-Copolymerisats gebildet. Das erstgenannte EVA-Copolymerisat ist durch einen VA-Anteil von 18 Gew.-%, bezogen auf die eingesetzte Gesamtmasse des Copolymeren, einen MFI von 2 g/10 min, gemessen nach DIN 53 735 bei 190°C mit einer Prüfbelastung von 2,16 kg, und eine Dichte von 0,926 g/cm³ charakterisiert. Das Propylenhomopolymerisats ist durch eine Dichte von 0,900 g/cm³ und einen MFI von 3,5 g/10 min, gemessen nach DIN 53 735 bei 230°C mit einer Prüfbelastung von 2,16 kg, charakterisiert. Das zweite EVA-Copolymerisat ist durch einen VA-Anteil von 5 Gew.-%, bezogen auf die eingesetzte Gesamtmasse des Copolymerisats, einen MFI von 1,8 g/10 min, gemessen nach DIN 53 735 bei 190°C mit einer Prüfbelastung von 2,16 kg, und eine Dichte von 0,926 g/cm³ charakterisiert. Diese Aussenschicht ist für die bevorzugte Verankerung von Adhäsiv-Klebstoffen eingestellt.

Die zweite 10 µm dicke Aussenschicht der Folie wurde aus einer Mischung unter Einsatz von 51 Gew.-% eines EVA-Copolymeren, charakterisiert durch einen VA-Anteil von 18 Gew.-%, bezogen auf die eingesetzte Gesamtmasse des Copolymeren, einem MFI von 2 g/10 min, gemessen nach DIN 53 735 bei 190°C mit einer Prüfbelastung von 2,16 kg, und einer Dichte von 0,926 g/cm³, 25 Gew. % eines Propylenhomopolymerisats, charakterisiert durch eine Dichte von 0,900 g/cm³ und einen MFI von 3,5 g/10 min, gemessen nach DIN 53 735 bei 230°C mit einer Prüfbelastung von 2,16 kg, 18 Gew.-% eines Ethylenpolymerisats, charakterisiert durch eine Dichte von 0,918 g/cm³ und einen MFI von 15 g/10 min, gemessen nach DIN 53 735 bei 190°C mit einer Prüfbelastung von 2,16 kg, sowie 6 Gew.-% Talkum zusammengesetzt.

### Beispiel 5

Mit einem Dreischicht-Blasfolienwerkzeug wurde eine farbige dreischichtige Folie mit einer Nennschichtdickenabfolge 15 µm, 50 µm, 15 µm geformt. Die Folie war beige eingefärbt.

Die 50 µm dicke Mittelschicht der Folie wurde zu 91 Gew.-% aus einem EVA-Copolymer mit einem VA-Anteil von 5 Gew.-%, einem Siliciumdioxid-Anteil von 1000 ppm, jeweils bezogen auf die Gesamtmasse des für die Mittelschicht verwendeten EVA-Copolymerisats, einem MFI von 1,8 g/10 min, gemessen nach DIN 53 735 bei 190°C mit einer Prüfbelastung von 2,16 kg, und einer Dichte von 0,926 g/cm³ gebildet. Zusätzlich enthielt sie 9 Gew.-% eines handelsüblichen beige-Masterbatches auf Polyethylen-Basis.

Die eine 10 µm dicke Aussenschicht der Folie wurde aus einer Mischung unter Einsatz von 55 Gew.-% eines EVA-Copolymeren, charakterisiert durch einen VA-Anteil von 18 Gew.-%, bezogen auf die eingesetzte Gesamtmasse des Copolymeren, einem MFI von 2 g/10 min, gemessen nach DIN 53 735 bei 190°C mit einer Prüfbelastung von 2,16 kg, und eine Dichte von 0,926 g/cm³, 25 Gew.-% eines Propylenhomopolymerisats, charakterisiert durch eine Dichte von 0,900 g/cm³ und einen MFI von 3,5 g/10 min, gemessen nach DIN 53 735 bei 230°C mit einer Prüfbelastung von 2,16 kg, sowie 11 Gew.-% eines EVA-Copolymeren, charakterisiert durch einen VA-Anteil von 5 Gew.-%, bezogen auf die eingesetzte Gesamtmasse des Copolymerisats, einen MFI von 1,8 g/10 min, gemessen nach DIN 53 735 bei 190°C mit einer Prüfbelastung von 2,16 kg, und eine Dichte von 0,926 g/cm³ gebildet. Zusätzlich enthielt diese Schicht 9 Gew.-% eines handelsüblichen beige-Masterbatches auf Polyethylen-Basis. Diese Aussenschicht eignet sich bevorzugt für die Verankerung von Adhäsiv-Klebstoffen.

Die zweite 10 µm dicke Aussenschicht der Folie wurde aus einer Mischung unter Einsatz von 51 Gew.-% eines EVA-Copolymeren, charakterisiert durch einen VA-Anteil von 18 Gew.-%, bezogen auf die eingesetzte Gesamtmasse des Copolymeren, einem MFI von 2 g/10 min, gemessen nach DIN 53 735 bei 190°C mit einer Prüfbelastung von 2,16 kg, und einer Dichte von 0,926 g/cm³, 25 Gew.-% eines Propylenhomopolymerisats, charakterisiert durch eine Dichte von 0,900 g/cm³ und einen MFI von 3,5 g/10 min, gemessen nach DIN 53 735 bei 230°C mit einer Prüfbelastung von 2,16 kg, 9 Gew.-% eines Ethylenpolymerisats, charakterisiert durch eine Dichte von 0,918 g/cm³ und einen MFI von 15 g/10 min, gemessen nach DIN 53 735 bei 190°C mit einer Prüfbelastung von 2,16 kg, sowie 6 Gew.-% Talkum zusammengesetzt. Zusätzlich enthielt diese Schicht 9 Gew.-% eines handelsüblichen beige-Masterbatches auf Polyethylen-Basis.

### Vergleichsbeispiel A

Auf einem Einschicht-Blasfolienwerkzeug wurde eine einschichtige Folie mit einer Nenndicke von 80 µm geformt. Als Ausgangsmaterial wurde eine Mischung aus 70 Gew.-% eines EVA-Copolymeren mit einem VA-Anteil von 5 Gew.-%, bezogen auf die eingesetzte Gesamtmasse EVA, einem MFI von 1,8 g/10 min, gemessen nach DIN 53 735 bei 190°C mit einer Prüfbelastung von 2,16 kg, und einer Dichte von 0,926 g/cm³ und 30 Gew.-% eines Propylenhomopolymerisats mit einer Dichte von 0,905 g/cm³ und einem MFI von 3,3 g/10 min, gemessen nach DIN 53 735 bei 230°C mit einer Prüfbelastung von 2,16 kg, eingesetzt.

### Vergleichsbeispiel B

Ein nach dem Stand der Technik bekannter zweischichtiger Aufbau wurde durch Beschichtung einer einschichtigen Folie mit einer Beschichtungsmasse auf einer mattierten Beschichtungswalze mit einer Prägetiefe von 16 µm erhalten. Die einschichtige Folie wurde aus einem EVA-Copolymer mit einem VA-Anteil von 5 Gew.-%, einem MFI von 1,8 g/10 min, gemessen nach DIN 53 735 bei 190°C mit einer Prüffielastung von 2,16 kg, und einer Dichte von 0,926 g/cm³ erzeugt. Sie wurde mittels eines Blasfolienwerkzeugs in einer Dicke von 55 µm hergestellt. Die Matt-Beschichtung erfolgte mit einem Polyethylen mit niedriger Dichte von 0,915 g/cm³ und einem MFI von 8 g/10 min, gemessen nach DIN 53 735 bei 190°C mit einer Prüfbelastung von 2,16 kg. Die Beschichtung erfolgte mit einer Nenndicke von 25 µm.

### Vergleichsbeispiel C

Mit einem Zweischicht-Blasfolienwerkzeug wurde eine zweischichtige Folie mit einer Nennschichtdickenabfolge 15 µm, 65 µm geformt. Die 65 µm dicke Schicht der Folie wurde aus einem EVA-Copolymer mit einem VA-Anteil von 5 Gew.-%, einem Siliciumdioxid-Anteil von 1000 ppm, jeweils bezogen auf die Gesamtmasse des für die Mittelschicht verwendeten EVA-Copolymerisats, einem MFI von 1,8 g/10 min, gemessen nach DIN 53 735 bei 190°C mit einer Prüfbelastung von 2,16 kg, und einer Dichte von 0,926 g/cm³ gebildet. Die 15 µm dicken Schichten der Folie wurde aus einem Blend bestehend aus 75 Gew.-% eines EVA-Copolymeren mit einem VA-Anteil von 18 Gew.-%, bezogen auf die für diese Schicht eingesetzte Gesamtmasse EVA, einem MFI von 2 g/10 min, gemessen nach DIN 53 735 bei 190°C mit einer Prüfbelastung von 2,16 kg, und einer Dichte von 0,926 g/cm³ und 25 Gew.-% eines Propylenhomopolymerisats mit einer Dichte von 0,905 g/cm³ und einem MFI von 3,3 g/10 min, gemessen nach DIN 53 735 bei 230°C mit einer Prüfbelastung von 2,16 kg, hergestellt.

Prüfungen an den im Rahmen der Beispiele und Vergleichsbeispiele hergestellten Mustern:

### Bestimmung der Glanzzahl

Die dimensionslose Glanzzahl wurde nach DIN 67 530 durch Reflektometermessung bestimmt.

### Bestimmung der Trübung

Die Trübung wurde ausschließlich an den nicht eingefärbten Proben nach ASTM D 1003 bestimmt.

### Bestimmung der Reißfestigkeit und -dehnung

Reißfestigkeit und -dehnung wurden nach DIN 53 544 ermittelt.

### Bestimmung der Rollneigung

Die Rollneigung wurde an Kreuzschnitten bestimmt. Hierzu wurden zwei 113 mm lange, sich unter einem Winkel von 90° schneidende Schnitte, die jeweils einen 45° Winkel mit der Maschinenlaufrichtung der Folie einschlossen und in ihren Abmessungen symmetrisch zum gegenseitigen Schnittpunkt sind, in die Proben eingebracht. Die Beurteilung erfolgte durch die Messung des größten Abstandes der sich einrollenden Spitzen des Kreuzschnittes.

Die nachfolgende Tabelle zeigt deutlich, daß die erfindungsgemäßen Folien aus den Beispielen 1 bis 5 den nach dem Stand der Technik bekannten Folien aus den Vergleichsbeispielen A bis C hinsichtlich der Verknüpfung der geforderten Materialeigenschaften überlegen sind.

Gegenüber der einschichtigen Vergleichsfolie (Vergleichsbeispiel A) besitzen die erfindungsgemäßen Folien deutlich verbesserte mechanische Festigkeiten. Sie besitzen im Vergleich zu den zweischichtigen Aufbauten (Vergleichsbeispiel B und C) eine verschwindend geringe Rollneigung. Ihre Oberflächen lassen sich bei Bedarf auch mit unterschiedlichen Glanzeigenschaften einstellen (Beispiele 3,4 und 5). Die Trübungswerte sind gegenüber den bekannten beschichteten Folien deutlich verringert.

**Tabelle 1**

| Eigenschaftsvergleich der in den Beispielen und Vergleichsbeispielen beschriebenen Folien | | | | | | | |
|---|---|---|---|---|---|---|---|
| | Glanzzahl 20° | Trübung | Reißfestigkeit längs | Reißfestigkeit quer | Reißdehnung längs | Reißdehnung quer | Rollneigung |
| Einheit | ./. | % | N/15mm | N/15mm | % | % | mm |
| Beispiel 1 | 1,7/1,7 | 57 | 22,1 | 21,7 | 678 | 746 | 0 |
| Beispiel 2 | 2,1/2,1 | 49 | 24,3 | 23,2 | 837 | 875 | 0 |
| Beispiel 3 | 2,3/1,7 *) | 60 | 18,6 | 17,7 | 678 | 731 | <1 |
| Beispiel 4 | 2,3/1,7 *) | 58 | 16,7 | 15,9 | 713 | 596 | <2 |
| Beispiel 5 | 2,4/1,6 *) | ./. | 23,5 | 22,8 | 698 | 659 | <1 |
| Vergleichsbeispiel A | 1,8/1,8 | 58 | 12,8 | 16,9 | 509 | 523 | 0 |
| Vergleichsbeispiel B | 1,2/49 **) | 75 | 15,1 | 20,2 | 533 | 264 | >5 |
| Vergleichsbeispiel C | 2,1/50 **) | 40 | 25,2 | 24,8 | 738 | 786 | >10 |

| | | | | | | | |
|---|---|---|---|---|---|---|---|
| *) Der jeweils niedrigere Werte bezieht sich auf die zusätzlich mit Schichtsilikat modifizierte Schicht/Seite der Folie. | | | | | | | |
| **) Der jeweils niedrigere Wert bezieht sich auf die mattierte Seite der Folie. | | | | | | | |

## Patentansprüche

1. Mindestens dreischichtige, beidseitig matte Folie mit geringer Rollneigung, dadurch gekennnzeichnet, daß die Folie durch Coextrusion hergestellt wurde, daß die Aussenschichten der Folie mindestens zwei verschiedene Polymerharze enthalten, wobei ein Harz ein Polyethylen oder Ethylencopolymerisat mit niedrigem Kristallinitätsgrad und einem Schmelzflußindex von 1 g/10 min bis 6 g/10min ist, gemessen nach DIN 53 735 bei 190°C mit einer Prüffielastung von 2,16 kg, bevorzugt sind Sauerstoffatome enthaltende Ethylencopolymerisate, eingesetzt mit Massenanteilen zwischen 60 Gew.-% und 85 Gew.-%, bezogen auf die Gesamtmasse der die Aussenschicht bildenden Materialien, und das zweite Polyolefinharz ein Propylenpolymerisat mit einem Schmelzflußindex von 1 g/10 min bis 6 g/10 min ist, gemessen nach DIN 53 735 bei 230°C und einer Prüfbelastung von 2,16 kg, bevorzugt sind Propylenhomopolymerisate, eingesetzt mit Anteilen von 15 Gew.-% bis 40 Gew.-%, bezogen auf die Gesamtmasse der die Aussenschicht bildenden Materialien, und die Kernschicht der Folie aus Polyethylen oder Ethylencopolymerisaten mit niedrigem Kristallinitätsgrad und einem Schmelzflußindex von 1 g/10 min bis 6 g/10min besteht, gemessen nach DIN 53 735 bei 190°C und einer Prüfbelastung von 2,16 kg bestehen, wobei Sauerstoffatome enthaltende Ethylencopolymerisate mit einem Comonomerrestanteil von 2 Gew.-% bis 10 Gew.-%, jeweils bezogen auf die für die Mittelschicht eingesetzte Gesamtpolymermasse, bevorzugt sind und die Oberflächen der erfindungsgemäßen Folien einen Glanzwert von höchstens 5, gemessen nach DIN 67 530 unter einem Winkel von 20°, aufweisen.

2. Folie nach Anspruch 1, dadurch gekennzeichnet, daß die Aussenschichten 20-30 Gew.-% eines partiell isotaktischen Propylenhomopolymerisats mit einer bevorzugten Dichte von 0,90 g/cm³ bis 0,91 g/cm³ und einem Schmelzflußindex von 2 g/10 min bis 5 g/10 min, besonders bevorzugt von 3 g/10 min bis 4 g/10 min, gemessen nach DIN 53 735 bei 230°C und einer Prüfbelastung von 2,16 kg, und 70-80 Gew.-% eines Ethylencopolymerisats mit einer Dichte zwischen 0,93 g/cm³ und 0,97 g/cm³, einem Anteil an Comonomerresten von 5 Gew.-% bis 25 Gew.-%, jeweils bezogen auf die Gesamtmasse des für die jeweilige Aussenschicht verwendeten jeweiligen Ethylencopolymerisats, und einem Schmelzflußindex von 1 g/10 min bis 3 g/10 min, gemessen nach DIN 53 735 bei 190°C und einer Prüfbelastung von 2,16 kg, wobei die Massenanteile der Polymerisate jeweils auf die Gesamtmasse der für die Herstellung der jeweiligen Aussenschicht eingesetzten Polymerrohstoffe bezogen sind, und das die Mittelschicht bildende Polymerharz ein Ethylencopolymerisat mit einer Dichte zwischen 0,91 g/cm³ und 0,94 g/cm³ und einem Schmelzflußindex von 1 g/10 min bis 3 g/10 min, gemessen nach DIN 53 735 bei 190°C und einer Prüfbelastung von 2,16 kg, bestehen.

3. Folie nach den Ansprüchen 1 und/oder 2, dadurch gekennzeichnet, daß einzelne oder alle Schichten der Folie weitere übliche Verarbeitungshilfsmittel und/oder Füllstoffe wie Silikate und/oder Wachse und/oder Stabilisatoren enthalten.

4. Folie nach wenigstens einem der vorangehenden Ansprüche, dadurch gekennzeichnet, daß wenigstens eine der Schichten Farbpigmente enthält.

5. Folie nach wenigstens einem der vorangehenden Ansprüche, dadurch gekennzeichnet, daß die Folie eine symmetrische Schichtdickenabfolge, bezogen auf den geometrischen Mittelpunkt der Folie, besitzt und der jeweilige Anteil der jeweiligen Aussenschicht an der Gesamtschichtdicke der Folie zwischen 10 % und 40 % und der Anteil der Mittelschicht zwischen 20 % und 80 %, jeweils bezogen auf die Gesamtdicke der Folie und besonders bevorzugt der summierte Anteil der Dicken der Aussenschichten an der Gesamtdicke der Folie nicht mehr als 50 % beträgt.

6. Folie nach einem der Ansprüche 1 bis 5, dadurch gekennzeichnet, daß die Folie durch einen Blasfolienprozeß hergestellt wurde.

7. Folie nach einem der Ansprüche 1 bis 6, dadurch gekennzeichnet, daß die Folie eine Gesamtschichtdicke von mindestens 40 µm und höchstens 200 µm, besonders bevorzugt mindestens 50 µm und höchstens 100 µm besitzt.

8. Folie nach wenigstens einem der vorangehenden Ansprüche, dadurch gekennzeichnet, daß die Folie ein- oder beidseitig wenigstens einem der bekannten chemischen und/oder physikalischen Oberflächen-Behandlungsverfahren unterzogen wurde.

9. Verwendung der Folie gemäß einem der Ansprüche 1 bis 8 als Trägerfilm für medizinische Pflaster.
